# EUROPÄISCHE PATENTSCHRIFT

(11) **EP 3 244 996 B1**
(45) Veröffentlichungstag und Bekanntmachung des Hinweises auf die Patenterteilung: **15.06.2022**
(21) Anmeldenummer: 15817901.0
(22) Anmeldetag: 29.12.2015
(51) Int. Cl.: B01J 23/52, B01J 35/00, B01J 35/08, B01J 37/02, C07C 67/00, C07C 69/54, B82Y 30/00

(54) **GOLD BASIERTEN KATALYSATOR FÜR DIE OXIDATIVE VERESTERUNG VON ALDEHYDEN ZU CARBONSÄUREESTERN**
CATALYST FOR OXIDATIVE ESTERIFICATION OF ALDEHYDES TO CARBOXYLIC ACID ESTERS
CATALYSEUR POUR L'ESTÉRIFICATION OXYDATIVE D'ALDÉHYDES EN ESTERS D'ACIDE CARBONIQUE

(30) Priorität: 16.01.2015 EP 15151466
(43) Veröffentlichungstag der Anmeldung: 22.11.2017
(73) Patentinhaber: Röhm GmbH, 64295 Darmstadt (DE)
(72) Erfinder: LYGIN, Alexander, 64347 Griesheim (DE); KRILL, Steffen, 64367 Mühltal (DE); GRÖMPING, Matthias, 64295 Darmstadt (DE); TEPPERIS, Andreas, 64732 Bad König (DE)
(74) Vertreter: Röhm Patent Association
(86) Internationale Anmeldenummer: PCT/EP2015/081338
(87) Internationale Veröffentlichungsnummer: WO 2016/113106

(56) Entgegenhaltungen:
- EP-A1- 2 177 267
- EP-A1- 2 210 664
- JP-A- 2007 296 429

## Beschreibung

### Gebiet der Erfindung

Die vorliegende Erfindung betrifft neuartige Katalysatoren für die oxidative Veresterung, mittels derer beispielsweise (Meth)acrolein zu Methyl(meth)acrylat umgesetzt werden kann. Die erfindungsgemäßen Katalysatoren zeichnen sich dabei insbesondere durch eine hohe mechanische und chemische Stabilität auch über sehr lange Zeiträume aus. Dies betrifft insbesondere eine Verbesserung gegenüber Katalysatoren des Standes der Technik, die in Medien mit auch geringem Wassergehalt im kontinuierlichen Betrieb relativ schnell Aktivität und/oder Selektivität verlieren.

### Stand der Technik

Die katalytische oxidative Veresterung von Aldehyden zur Herstellung von Carbonsäureestern ist im Stand der Technik umfänglich beschrieben. So kann man z.B. derart sehr effizient Methylmethacrylat aus Methacrolein (MAL) und Methanol herstellen. Insbesondere in US 5,969,178 und in US 7,012,039 wird ein Verfahren zur kontinuierlichen Herstellung von MMA aus Isobuten oder tert-Butanol beschrieben. Dabei weist das Verfahren die folgenden Schritte auf: 1) Oxidation von Isobuten oder tert-Butanol zu Methacrolein und 2) Direkte oxidative Veresterung von MAL mit Methanol zu MMA mit einem Pd-Pb-Katalysator, der sich auf einem oxidischen Träger befinden.

Jedoch weisen sämtliche aus dem Stand der Technik bekannten Katalysatoren bei längeren Standzeiten eine Empfindlichkeit insbesondere gegenüber wässrigen Medien auf. So werden zum Beispiel in EP 1 393 800 zwar gute Aktivitäten und Selektivitäten beschreiben, es wird jedoch gleichzeitig keine Auskunft über die Lebensdauer der Katalysatoren gegeben. Es handelt sich dabei um goldhaltige Katalysatoren, insbesondere um Goldnanopartikel mit einem durchschnittlichen Durchmesser von weniger als 6 nm auf einem Träger. Die Selektivitäten zu MMA werden bei einem Gehalt von 4,5 Gew% Au mit bis zu 93% und die Raum-Zeit-Ausbeute mit bis zu 50,7 mol MMA/kg Kat*h angegeben. Der pH-Wert der goldhaltigen Lösung bei der Katalysatorherstellung liegt im Bereich von 5 bis 10, bevorzugt zwischen 6 und 9.

In der US 6,040,472 werden alternative Katalysatoren, die jedoch im Vergleich nur zu unzureichenden Aktivitäten und Selektivitäten zu MMA führen, beschrieben. Es handelt sich in diesem Fall um Pd-Pbhaltige Katalysatoren mit Schalenstruktur. Die Selektivitäten zu MMA werden mit bis zu 91 % und die Raum-Zeit-Ausbeute mit bis zu 5,3 mol angegeben.

In EP 2 177 267 und EP 2 210 664 werden nickelhaltige Katalysatoren mit Schalenstruktur beschrieben. Selektivität zu MMA beträgt bei diesen Katalysatoren bis zu 97%. Die Raum-Zeit-Ausbeute wird mit 9,7 mol MMA/kg Kat*h bei einem Goldanteil im Katalysator von ca. 1 Gew% beschrieben.

US 2013/0172599 wiederum beschreibt ein siliziumhaltiges Material bestehend aus Si, Al und einer basischen dritten Komponente sowie einem Metall mit erhöhter Säurebeständigkeit. Dabei handelt es sich um Ni, Co, Zn oder Fe. Dieses Material kann als Träger für Edelmetall-haltige Katalysatoren eingesetzt werden. Eine bevorzugte Katalysatorvariante für die oxidative Veresterung von Methacrolein zu MMA beinhaltet einen Au/NiO Katalysator geträgert auf SiO₂-Al₂O₃-MgO.

Jedoch geht aus dem Vergleichsbeispiel 2 hervor, dass dieser Katalysator mit der Zeit Aktivität verliert. Dies ist wahrscheinlich auf die negative Auswirkung von im Reaktionsgemisch vorhandenem Wasser zurückzuführen.

JP 2007 296429 A offenbart einen Katalysator in Form von Partikeln für die oxidative Veresterung von Aldehyden zu Carbonsäureestern.

### Aufgabe

Aufgabe der vorliegenden Erfindung war primär, die Herstellung eines neuartigen Katalysators für eine hoch selektive oxidative Veresterung von Aldehyden zu Carbonsäureester zur Verfügung zu stellen. Dabei soll dieser Katalysator eine hohe mechanische und chemische Stabilität, insbesondere in Wasser- und Carbonsäure-haltigen Gemischen, aufweisen und über einen gegenüber dem Stand der Technik längeren Erhalt der Aktivität (Produktivität) und der Selektivität verfügen.

Insbesondere bestand die Aufgabe, dass dieser Katalysator für die oxidative Veresterung von Methacrolein zu einem Alkylmethacrylat, insbesondere zu MMA geeignet sein soll.

Weitere nicht explizit genannte Aufgaben können sich aus der Beschreibung, den Beispielen, den Ansprüchen oder dem Gesamtzusammenhang der vorliegenden Erfindung ergeben.

### Lösung

Gelöst wurden die gestellten Aufgaben mit Hilfe neuartiger Hydrolyse-resistenter Katalysatoren. Diese erfindungsgemäßen Katalysatoren liegen in Form von Partikeln vor und können über einen langen Zeitraum in der oxidativen Veresterung von Aldehyden zu Carbonsäureestern eingesetzt werden. Die erfindungsgemäßen Katalysatoren sind dabei dadurch gekennzeichnet, dass sie mit Ausnahme des Sauerstoffs aus den folgenden Komponenten a) bis d) bestehen:
a) 0,01 bis 10 mol%, bevorzugt 0,05 bis 2 mol% Gold,
b) 40 bis 94 mol% Silizium,
c) 3 bis 40 mol% Aluminium und
d) 2 bis 40 mol%, bevorzugt 2 bis 30 mol% mindestens eines weiteren Elements ausgewählt aus Alkalimetallen, Erdalkalimetallen, Metalle der seltenen Erden, Ti, Zr, Cu, Mn, Pb, Sn oder Bi, dabei liegen die Komponenten b) bis d) als Oxide vor. Die aufgeführten Mengenangaben der Komponenten a) bis d) beziehen sich auf 100 mol% der Zusammensetzung des Katalysators, ohne dass dabei Sauerstoff einbezogen wäre. Diese Angabe der Zusammensetzung unter nicht Berücksichtigung des in den Oxiden vorhandenen Sauerstoffs ist zweckmäßig, da einige der Elemente deutlich unterschiedliche Oxidationsstufen aufweisen bzw. beispielsweise auch Mischoxide vorliegen können.

Weiterhin weist der Katalysator eine Schalenstruktur aus einem Kern und mindestens einer Schale, bevorzugt aus einem Kern und einer oder zwei Schalen auf. Dabei ist Komponente a) zu mindestens 80 % der Gesamtmasse der Komponente a), bevorzugt zu mindestens 90 % und besonders bevorzugt vollständig Bestandteil einer Schale.

In einer bevorzugten Variante weist der Katalysator neben dem Kern und einer direkt darauf aufliegenden goldhaltigen Schale eine weitere, dünne äußere Schale auf, die insbesondere aus einer oder mehreren Komponenten b) bis d) besteht und nur maximal geringe Mengen der Komponente a) aufweist. Diese Schale dient zu einer weiteren Verlängerung der Standzeit durch die Reduktion von Abrieb der Komponente a) bei hoher mechanischer Belastung.

Schalenstruktur bedeutet in diesem Fall, dass der Kern an sich eine mehr oder weniger in sich homogene Struktur aufweist, während die Schale, d.h. ein den Kern umschließender Bereich, eine davon unterscheidbare Struktur hat. "Mehr oder weniger homogen" bedeutet dabei, dass es innerhalb des Kerns oder einer Schale durchaus lokale Unterschiede, z.B. in Form von oxidischen Nanokristallen, geben kann, dass jedoch über den gesamten Kern bzw. die gesamte Schale solche Nanophasen gleichmäßig verteilt sind. Als Beispiel sei dazu das Gold in der katalytisch aktiven Schale genannt, welches in Form von Nanopartikeln gleichmäßig über diese Schale verteilt ist.

Insbesondere ist mit Schalenstruktur eine solche Verteilung der Aktivkomponente (Au) im ganzen Katalysatorkörper gemeint, bei der der Großteil der Goldatome sich nah zu der Oberfläche, und damit in einem Bereich, der von außen gesehen weniger als 50% des Partikelradius ausmacht, befindet und damit nicht gleichmäßig über den gesamten Katalysatorkörper verteilt ist.

Auch sei darauf hingewiesen, dass die Grenze zwischen dem Kern und einer Schale, bzw. zwischen zwei Schalen nicht scharf sein muss, sondern durchaus in Form eines Gradienten mit sich ändernder Zusammensetzung vorliegen kann. So kann beispielsweise die Konzentration an Goldnanopartikeln vom Kern aus gesehen von innen nach außen zunehmen. Dies ergibt sich allein schon aus der Tatsache, dass die erfindungsgemäßen Partikel in der Regel eine relativ hohe Porosität aufweisen. Dabei befinden sich bei weniger bevorzugten Ausführungsformen mit mehr als einer Schale, wobei die Goldpartikel in einer äußeren Schale konzentriert sind und ein Gradient der Goldpartikelverteilung vorliegt, erfindungsgemäß weniger als 20%, bevorzugt weniger als 10% und besonders bevorzugt keine Goldpartikel in den inneren mindestens 50% des Gesamtpartikelradius, wobei dieser neben dem Kern auch innere, goldarme bzw. -freie Schalen umfassen kann.

Ein weiteres essentielles Merkmal der erfindungsgemäßen Katalysatoren ist, dass diese einen Point of Zero Charge (bzw. "Punkt der Null-Ladung", im Weiteren als PZC-Wert bezeichnet) zwischen 7 und 11 aufweisen. Eine genaue Definition und Methoden zur Bestimmung des PZC-Wertes findet sich beispielsweise in Powder technology, 103, 1999, 30-36. Hier wird ausgelegt, dass der PZC-Wert eines der wichtigsten Charakteristika einer oxidischen Oberfläche eines Feststoffs, der sich in einem flüssigen Medium befindet, darstellt. Der PZC-Wert kann dabei als Analoga zu dem pH-Wert einer Flüssigkeit, insbesondere eines wässrigen Mediums angesehen werden, wenn die Summe der negativen und positiven Ladungen an der oxidischen Oberfläche ausgeglichen sind. So ist die Ladung einer oxidischen Oberfläche negativ, wenn der pH-Wert der umgebenden Flüssigkeit größer ist als der PZC-Wert und umgekehrt ist die Oberflächenladung positiv, wenn der PZC-Wert größer ist als der pH-Wert des umgebenden flüssigen Mediums. Geeignete Methoden zur Bestimmung des PZC-Werts, insbesondere für Partikel mit einem Durchmesser kleiner 20 µm sind die Bestimmung eines Zeta Potentials und die elektrophoretische Mikromobilität. Die Bestimmung kann alternativ, und deutlich einfacher und unabhängig von der Partikelgröße auch durch einfache Bestimmung des pH-Werts des umgebenden, in der Regel wässrigen Mediums, in dem die oxidischen Partikel konzentriert suspendiert sind, erfolgen. Auf diese Weise erhält man einen angenäherten, für die Durchführung der vorliegenden Erfindung ausreichenden PZC-Wert, mittels dem man unterschiedliche Partikel gut miteinander vergleichen kann.

Mit dieser Erfindung hat sich überraschenderweise gezeigt, dass insbesondere eine bestimmte Kombination von Gold als Komponente a), Oxiden des Siliziums als Komponente b) und des Aluminiums als Komponente c), sowie einem oder mehreren Oxiden ausgewählt bevorzugt aus den folgenden Elementen Li, Na, K, Rb, Cs, Be, Mg, Ca, Sr, Ba, Sc, Y, Ti, Zr, Cu, Mn, Pb, Sn, Bi oder Lanthanoiden mit den Ordnungszahlen 57 bis 71 als Komponente d) die oben genannten Aufgaben löst. Dabei werden überraschenderweise vor allem der Erhalt von Aktivität und Selektivität des für die oxidative Veresterung eingesetzten Katalysators über eine lange Zeit ermöglicht, ohne dass diese merklich reduziert würden. Besonders bevorzugt handelt es sich bei Komponente d) um Mg, Ce, La, Y, Zr, Mn, Pb, Bi und/oder Mischungen aus diesen Elementen.

Dabei wurde insbesondere überraschend gefunden, dass die Acidität/Basizität des Katalysators eine entscheidende Rolle fürs Erreichen der besten Katalysatorperformance spielt. Ist der Katalysator zu sauer, bilden sich im Reaktionsgemisch aus den Aldehyden und Alkoholen, also den Rohstoffen der oxidativen Veresterung, vermehrt Acetale als Nebenprodukt und die Selektivität wird damit deutlich gesenkt. Ist der Katalysator demgegenüber zu basisch, so bilden sich im Reaktionsgemisch die Michael-Addukte von Alkoholen mit Doppelbindungen, wie sie beispielsweise im Methacrolein (MAL) vorhanden sind, und die Selektivität wird damit ebenfalls deutlich gesenkt. Der Katalysator braucht erfindungsgemäß eine etwas basische Oberfläche. Als Maß für die Acidität/Basizität einer Katalysatoroberfläche kann deren PZC-Wert genommen werden.

Bevorzugt haben die Katalysator-Partikel einen mittleren Durchmesser zwischen 10 und 200 µm, besonders bevorzugt zwischen 20 und 120 µm und dabei eine sphärische Form. Gleichzeitig oder unabhängig davon liegt die Komponente a) bevorzugt in Form von Partikeln mit einem mittleren Durchmesser zwischen 2 und 10 nm vor.

Neben den beschriebenen Katalysatoren für eine oxidative Veresterung ist insbesondere auch ein Verfahren zur Herstellung von Katalysatoren für eine oxidative Veresterung Bestandteil der vorliegenden Erfindung. Dieses Verfahren zur Herstellung von Katalysator-Partikeln ist dadurch gekennzeichnet, dass ein basischer Mischoxid-Träger mit einem PZC-Wert zwischen 8 und 12 und einem mittleren Durchmesser zwischen 10 und 200 µm, bestehend aus b) 40 bis 94 mol% Silizium, c) 3 bis 40 mol% Aluminium und d) 2 bis 40 mol% mindestens eines weiteren Elements ausgewählt aus Alkalimetallen, Erdalkalimetallen, Metalle der seltenen Erden, Ti, Zr, Cu, Mn, Pb, Sn oder Bi, wobei die Komponenten b) bis d) als Oxide vorliegen und sich die Mengenangaben der Komponenten a) bis d) auf 100 mol% der Zusammensetzung des Katalysators ohne Sauerstoff beziehen, mit einer goldhaltigen, die Komponente a) bildenden Lösung, aufweisend einen pH-Wert zwischen 0,5 und 5,0 versetzt wird. Bei der goldhaltigen Lösung kann es sich insbesondere um eine wässrige Lösung von Tetrachloridogoldsäure handeln. Diese Lösung kann optional auch lösliche Salze anderer Elementen aus b), c) und/oder d) enthalten.

Die Art der Auftragung der goldhaltigen Aktivkomponente auf den Träger aus den Komponenten b), c) und d) ist erfindungsgemäß entscheidend. Dabei spielen sowohl die Basizität des angewendeten Trägers, als auch die Acidität der goldhaltigen Lösung eine große Rolle. Die Kombination aus diesen zwei Faktoren ermöglicht es, einen Katalysator mit Schalenstruktur zu synthetisieren, der hohe Aktivität, Selektivität, Hydrolysestabilität und lange Standzeiten aufweist.

Die Azidität der goldhaltigen Lösung hat vor allem einen großen Einfluss auf die Ausbildung diverser Spezies von Gold(III)-Komplexionen in der Lösung (siehe dazu beispielsweise Geochimica et Cosmochimica Acta Vol. 55, pp. 671-676) und letztendlich auf die Natur der Bindung an die Oberfläche des Trägers. In vielen Literaturstellen wird ein pH-Bereich der goldhaltigen Lösung um die 7 bevorzugt (siehe z.B. EP 1 393 800). Erfindungsgemäß wurde überraschend gefunden, dass der pH-Bereich zwischen 0,5 und 5,0 sich als beste Bedingung für die Katalysatorperformance des mittels dieses Verfahrens hergestellten Katalysators zeigte.

In einer besonderen Variante dieses Verfahrens enthält die goldhaltige Lösung neben der Goldverbindung mindestens eine zusätzliche Verbindung. Diese Verbindung weist wiederum Komponenten b), c) und/oder d) in ionischer Form auf. Mit dieser Variante kann eine zusätzliche Schutzschicht für Goldnanopartikeln entstehen, die für höhere Standzeit des Katalysators zusätzlich förderlich ist. Komponente b) in ionischer Form bedeutet in diesem Fall, dass Silikate, z.B. Natriumsilikate oder Ammoniumsilikate, die später bei einem optionalen thermischen Sintern oder Kalzinieren zu Siliziumoxiden umgesetzt werden, in der Lösung enthalten sind.. Komponenten c) oder d) in ionischer Form bedeuten die entsprechenden wasserlöslichen Salze, z.B. Aluminiumnitrat, Aluminiumsulfat usw.

Nach der beschriebenen Herstellung der Partikel, einem Isolieren, z.B. mittels Filtration, und weiterem Reinigen, werden diese abschließend besonders bevorzugt Kalziniert. Dies kann beispielsweise bei Temperaturen zwischen 300 und 600 °C geschehen.

Neben den beschriebenen Katalysatoren und dem beschriebenen Verfahren zu deren Herstellung ist auch die Verwendung eines solchen erfindungsgemäßen, bzw. erfindungsgemäß hergestellten Katalysators bei der Umsetzung von (Meth)acrolein mit Sauerstoff und einem monofunktionellen Alkohol zu einem Alkyl(meth)acrylat Bestandteil der vorliegenden Erfindung. Die Klammer in (Meth)acrolein bedeutet dabei, dass es sich bei diesem Rohstoff sowohl um Acrolein als auch um Methacrolein handeln kann. Entsprechend bedeutet Alkyl(meth)acrylat sowohl Alkylacrylat, als auch Alkylmethacrylat. Dabei wird diese Alkylgruppe durch den eingesetzten Alkohol bestimmt.

Bevorzugt wird bei dieser Verwendung Methacrolein in Anwesenheit des erfindungsgemäßen Katalysators mit Sauerstoff und Methanol zu MMA umgesetzt.

Alternativ kann bei dieser Verwendung auch (Meth)acrolein mit Sauerstoff und einem di-, tri- oder tetra-funktionellen Alkohol zu einem Hydroxyalkyl(meth)acrylat und di-, tri- bzw. tetra-(Meth)acrylat umgesetzt werden. Die letzteren Verbindungen sind als Vernetzer bekannt. Ein besonders bevorzugtes Beispiel für einen di-funktionellen Alkohol ist Ethylenglycol.

Besonders bevorzugt wird die oxidative Veresterung in Anwesenheit des erfindungsgemäßen Katalysators kontinuierlich durchgeführt. Ganz besonders bevorzugt wird der Katalysator während der oxidativen Veresterung in einem gerührten Reaktor in Suspensionsform (als Slurry) angewendet.

### Beispiele

### PZC Messung

Pulvermaterial (3,0 g) wird in 7,5 mL einer 0,1 gew%igen Lösung von NaCl in VE Wasser suspendiert und mit einem Magnetrührer gerührt (100 rpm). Der pH-Wert dieser Suspension wird nach 15 min Rühren bei Raumtemperatur mit einer pH Sonde gemessen. Dieser Wert wird weiterhin als PZC bezeichnet.

### Herstellung eines prä-formierten Trägers

### Beispiel 1

In einem 250 mL Becherglas werden 21,35 g Mg(NO₃)₂*6H₂O und 31,21 g Al(NO₃)₃*9H₂O vorgelegt und in 41,85 g VE Wasser unter Rühren auf einem Magnetrührer gelöst. Danach werden 1,57 g 60 gew%ige HNO₃ unter Rühren zugegeben. 166,67 g Silicasol (Köstrosol 1530AS von der Firma Bad Köstritz) werden in einem 500 mL Dreihalskolben eingewogen und unter Rühren auf 15 °C gekühlt. 2,57 g 60 gew%ige HNO₃ werden unter weiterem Rühren langsam zum Sol zugegeben. Bei 15 °C wird die Nitratlösung innerhalb von 45 min zum Sol unter Rühren zugegeben. Nach der Zugabe wird das Gemisch innerhalb von 30 min auf 50 °C erhitzt und für weitere 24 h bei dieser Temperatur gerührt. Nach dieser Zeit wird das Gemisch in einer dünnen Schicht auf einer vorgeheizten Schale bei 130 °C im Trockenschrank getrocknet und anschließend zu einem feinen Pulver gemörsert. Das getrocknete Pulver wird in dünner Schicht im Naberofen über 2h auf 300 °C erhitzt, 3h bei 300 °C gehalten, über 2h auf 600 °C erhitzt und weitere 3h bei 600 °C gehalten. PZC von diesem Material war 9,81.

### Beispiel 2

In einem 250 mL Becherglas werden 32,03 g Mg(NO₃)₂*6H₂O, 31,21 g Al(NO₃)₃*9H₂O zusammen vorgelegt und in 41,85 g VE Wasser unter Rühren mit einem Magnetrührer gelöst. Danach werden 1,57 g 60 gew%ige HNO₃ unter Rühren zugegeben. 166,67 g Silicasol (Köstrosol 1530AS von der Firma Bad Köstritz) werden in einem 500 mL Dreihalskolben eingewogen und unter Rühren auf 15 °C gekühlt. 2,57 g 60 gew%ige HNO₃ werden bei Rühren langsam zum Sol zugegeben. Bei 15 °C wird die Nitratlösung über 45 min zum Sol unter Rühren zugegeben. Nach der Zugabe wird das Gemisch innerhalb von 30 min auf 50 °C erhitzt und für weitere 24 h bei dieser Temperatur gerührt. Nach dieser Zeit wird das Gemisch in dünner Schicht auf einer vorgeheizten Schale bei 130 °C im Trockenschrank getrocknet, anschließend zu einem feinen Pulver gemörsert. Das getrocknete Pulver wird in dünner Schicht im Naberofen innerhalb von 2 h auf 300 °C erhitzt, 3h bei 300 °C gehalten, innerhalb von weiteren 2h auf 600 °C erhitzt und schließlich für weitere 3 h bei 600 °C gehalten. PZC von diesem Material war 9,36.

### Beispiel 3

In einem 250 mL Becherglas werden 10,68 g Mg(NO₃)₂*6H₂O und 31,21 g Al(NO₃)₃*9H₂O zusammen vorgelegt und in 41,85 g VE Wasser unter Rühren mit einem Magnetrührer gelöst. Danach werden 1,57 g 60 gew%ige HNO₃ unter Rühren zugegeben. 166,67 g Silicasol (Köstrosol 1530AS von der Firma Bad Köstritz) werden in einem 500 mL Dreihalskolben eingewogen und unter Rühren auf 15 °C gekühlt. 2,57 g 60 gew%ige HNO₃ werden unter Rühren langsam zum Sol zugegeben. Bei 15 °C wird die Nitratlösung innerhalb von 45 min unter Rühren zum Sol zugegeben. Nach der Zugabe wird das Gemisch innerhalb von 30 min auf 50 °C erhitzt und für weitere 24 h bei dieser Temperatur gerührt. Nach dieser Zeit wird das Gemisch in dünner Schicht auf einer vorgeheizten Schale bei 130 °C im Trockenschrank getrocknet, anschließend zu einem feinen Pulver gemörsert. Das getrocknete Pulver wird in dünner Schicht im Naberofen über 2 h auf 300 °C erhitzt, 3 h bei 300 °C gehalten, innerhalb weiterer 2h auf 600 °C erhitzt und schließlich weitere 3h bei 600 °C gehalten. PZC von diesem Material war 7,91.

### Herstellung eines dotierten Trägers

### Beispiele 4 bis 13, sowie Vergleichsbeispiel 1

### Vergleichsbeispiel 1 (Sn)

SnCl₂*2H₂O (440 mg, 1,95 mmol) wurden in 5 g destilliertem Methanol gelöst und die Lösung mit 10 g des vorher beschriebenen, prä-formierten SiO₂-Al₂O₃-MgO Trägers aus Beispiel 1 vermischt und dabei intensiv geschüttelt. Der so erhaltene trocken aussehende Träger wurde in einer dünnen Schicht im Trockenschrank bei 105 °C 10 h getrocknet, dann fein gemörsert und in dünner Schicht im Naberofen innerhalb von 2 h auf 300 °C erhitzt, 3h bei 300 °C gehalten, innerhalb weiterer 2 h auf 600 °C erhitzt und schließlich für weitere 3 h bei 600 °C gehalten. PZC von diesem Material war 6,85.

### Beispiel 4 (Bi)

Bi(NO₃)₃*5H₂O (947 mg, 1,95 mmol) wurden in 5 g VE Wasser unter Zugabe von 2,9 g 60 gew%iger HNO₃ gelöst und die Lösung mit 10 g des vorher beschriebenen, prä-formierten SiO₂-Al₂O₃-MgO Trägers aus Beispiel 1 vermischt und dabei intensiv geschüttelt. Der so erhaltene trocken aussehende Träger wurde in einer dünnen Schicht im Trockenschrank bei 105 °C 10h getrocknet, dann fein gemörsert und in dünner Schicht im Naberofen innerhalb von 2 h auf 300 °C erhitzt, 3 h bei 300 °C gehalten, innerhalb von 2 h auf 600 °C erhitzt und für weitere 3 h bei 600 °C gehalten. PZC von diesem Material war 9,21.

### Beispiel 5 (Mn)

Mn(NO₃)₂*4H₂O (494 mg, 1,95 mmol) wurden in 5 g VE Wasser gelöst und die Lösung mit 10 g des vorher beschriebenen, prä-formierten SiO₂-Al₂O₃-MgO Trägers aus Beispiel 1 vermischt, intensiv geschüttelt. Der so erhaltene trocken aussehende Träger wurde in einer dünnen Schicht im Trockenschrank bei 105 °C über 10 h getrocknet, dann fein gemörsert und in dünner Schicht im Naberofen innerhalb von 2 h auf 300 °C erhitzt, 3h bei 300 °C gehalten, innerhalb von 2h auf 600 °C erhitzt und für weitere 3h bei 600 °C gehalten. PZC von diesem Material war 8,95.

### Beispiel 6 (Ce)

Ce(NO₃)₃*6H₂O (848 mg, 1,95 mmol) wurden in 5 g VE Wasser gelöst und die Lösung mit 10 g des vorher beschriebenen, prä-formierten SiO₂-Al₂O₃-MgO Trägers aus Beispiel 1 vermischt und dabei intensiv geschüttelt. Der so erhaltene trocken aussehende Träger wurde in einer dünnen Schicht im Trockenschrank bei 105 °C 10h getrocknet, dann fein gemörsert und in dünner Schicht im Naberofen innerhalb von 2 h auf 300 °C erhitzt, 3 h bei 300 °C gehalten, innerhalb von 2 h auf 600 °C erhitzt und schließlich 3 h bei 600 °C gehalten. PZC von diesem Material war 9,56.

### Beispiel 7 (Zr)

ZrO(NO₃)₂*6H₂O (663 mg, 1,95 mmol) wurden in 5 g VE Wasser gelöst und die Lösung mit 10 g des vorher beschriebenen, prä-formierten SiO₂-Al₂O₃-MgO Trägers aus Beispiel 1 vermischt und dabei intensiv geschüttelt. Der so erhaltene trocken aussehende Träger wurde in einer dünnen Schicht im Trockenschrank bei 105 °C 10h getrocknet, dann fein gemörsert und in dünner Schicht im Naberofen innerhalb von 2 h auf 300 °C erhitzt, 3 h bei 300 °C gehalten, innerhalb von 2 h auf 600 °C erhitzt und schließlich 3 h bei 600 °C gehalten. PZC von diesem Material war 8,90.

### Beispiel 8 (Pb)

Pb(NO₃)₂ (647 mg, 1,95 mmol) wurden in 5 g VE Wasser gelöst und die Lösung mit 10 g des vorher beschriebenen, prä-formierten SiO₂-Al₂O₃-MgO Trägers aus Beispiel 1 vermischt und dabei intensiv geschüttelt. Der so erhaltene trocken aussehende Träger wurde in einer dünnen Schicht im Trockenschrank bei 105 °C 10h getrocknet, dann fein gemörsert und in dünner Schicht im Naberofen innerhalb von 2 h auf 300 °C erhitzt, 3 h bei 300 °C gehalten, innerhalb von 2 h auf 600 °C erhitzt und schließlich 3 h bei 600 °C gehalten. PZC von diesem Material war 9,35.

### Beispiel 9 (AI)

Al(NO₃)₃*9H₂O (734 mg, 1,95 mmol) wurden in 5 g VE Wasser gelöst und die Lösung mit 10 g des vorher beschriebenen, prä-formierten SiO2-Al2O3-MgO Trägers aus Beispiel 1 vermischt, intensiv geschüttelt. Der so erhaltene trocken aussehende Träger wurde in einer dünnen Schicht im Trockenschrank bei 105 °C 10h getrocknet, dann fein gemörsert und in dünner Schicht im Naberofen in 2h auf 300 °C erhitzt, 3h bei 300 °C gehalten, in 2h auf 600 °C erhitzt, 3h bei 600 °C gehalten. PZC von diesem Material war 9,12.

### Beispiel 10 (Y)

Y(NO₃)₃*6H₂O (750 mg, 1,95 mmol) wurden in 5 g VE Wasser gelöst und die Lösung mit 10 g des vorher beschriebenen, prä-formierten SiO₂-Al₂O₃-MgO Trägers aus Beispiel 1 vermischt und dabei intensiv geschüttelt. Der so erhaltene trocken aussehende Träger wurde in einer dünnen Schicht im Trockenschrank bei 105 °C 10h getrocknet, dann fein gemörsert und in dünner Schicht im Naberofen innerhalb von 2 h auf 300 °C erhitzt, 3 h bei 300 °C gehalten, innerhalb von 2 h auf 600 °C erhitzt und schließlich 3 h bei 600 °C gehalten. PZC von diesem Material war 9,34.

### Beispiel 11 (La)

La(NO₃)₃*6H₂O (845 mg, 1,95 mmol) wurden in 5 g VE Wasser gelöst und die Lösung mit 10 g des vorher beschriebenen, prä-formierten SiO2-Al₂O₃-MgO Trägers vermischt und dabei intensiv geschüttelt. Der so erhaltene trocken aussehende Träger wurde in einer dünnen Schicht im Trockenschrank bei 105 °C 10h getrocknet, dann fein gemörsert und in dünner Schicht im Naberofen innerhalb von 2 h auf 300 °C erhitzt, 3 h bei 300 °C gehalten, innerhalb von 2 h auf 600 °C erhitzt und schließlich 3 h bei 600 °C gehalten. PZC von diesem Material war 10,20.

### Beispiel 12 (Mg)

Mg(NO₃)₂*6H₂O (2,0 g, 7,8 mmol) wurden in 5 g VE Wasser gelöst und die Lösung mit 10 g des vorher beschriebenen, prä-formierten SiO2-Al₂O₃-MgO Trägers aus Beispiel 1 vermischt und dabei intensiv geschüttelt. Der so erhaltene trocken aussehende Träger wurde in einer dünnen Schicht im Trockenschrank bei 105 °C 10h getrocknet, dann fein gemörsert und in dünner Schicht im Naberofen innerhalb von 2 h auf 300 °C erhitzt, 3 h bei 300 °C gehalten, innerhalb von 2 h auf 600 °C erhitzt und schließlich 3 h bei 600 °C gehalten. PZC von diesem Material war 12,14.

### Beispiel 13 (Li)

LiOH (470 mg, 19,5 mmol) wurden in 5 g VE Wasser gelöst und die Lösung mit 10 g des vorher beschriebenen, prä-formierten SiO₂-Al₂O₃-MgO Trägers aus Beispiel 1 vermischt und dabei intensiv geschüttelt. Der so erhaltene trocken aussehende Träger wurde in einer dünnen Schicht im Trockenschrank bei 105 °C 10h getrocknet, dann fein gemörsert und in dünner Schicht im Naberofen innerhalb von 2 h auf 300 °C erhitzt, 3 h bei 300 °C gehalten, innerhalb von 2 h auf 600 °C erhitzt und schließlich 3 h bei 600 °C gehalten. PZC von diesem Material war 12,31.

### Katalysatorherstellung (Auftragen von Au auf einen dotierten Träger)

### Beispiele 14 bis 27, sowie Vergleichsbeispiele 2 bis 5

### Vergleichsbeispiel 2

Eine Suspension von 10 g dotierten Träger aus Vergleichsbeispiel 1 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 8,89.

### Beispiel 14

Eine Suspension von 10 g dotierten Träger aus Beispiel 4 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 9,09.

### Beispiel 15

Eine Suspension von 10 g dotierten Träger aus Beispiel 5 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 8,72.

### Beispiel 16

Eine Suspension von 10 g dotierten Träger aus Beispiel 6 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 8,92.

### Beispiel 17

Eine Suspension von 10 g dotierten Träger aus Beispiel 7 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 8,87.

### Beispiel 18

Eine Suspension von 10 g dotierten Träger aus Beispiel 8 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 8,59.

### Beispiel 19

Eine Suspension von 10 g dotierten Träger aus Beispiel 9 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 8,82.

### Vergleichsbeispiel 3

Eine Suspension von 10 g dotierten Träger aus Beispiel 1 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 12,0, eingestellt durch Zugabe von 20 wt% NaOH) zugegeben. Nach der Zugabe wurde das Gemisch weitere 30 min gerührt, dann abgekühlt und auf Raumtemperatur abgekühlt. 1,0 g NaBH₄ wurde bei RT zugegeben, das Reaktionsgemisch weitere 30 min gerührt und dann filtriert. Anschließend wurde sechsmal mit jeweils 50 mL Wasser gewaschen. Das Material wurde 10 h bei 105 °C getrocknet. PZC von diesem Material war 11,15.

### Beispiel 20

Eine Suspension von 10 g dotierten Träger aus Beispiel 1 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 9,49.

### Beispiel 21

Eine Suspension von 10 g dotierten Träger aus Beispiel 2 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 8,57.

### Vergleichsbeispiel 4

Eine Suspension von 10 g dotierten Träger aus Beispiel 3 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 6,99.

### Beispiel 22

Eine Suspension von 10 g dotierten Träger aus Beispiel 1 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 0,4, eingestellt durch Zugabe von konzentrierter Salzsäure) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 9,29.

### Beispiel 23

Eine Suspension von 10 g dotierten Träger aus Beispiel 1 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 5,2, eingestellt durch Zugabe von 20 gew%iger NaOH-Lösung) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 9,63.

### Beispiel 24

Eine Suspension von 10 g dotierten Träger aus Beispiel 10 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 8,80.

### Vergleichsbeispiel 5

Eine Suspension von 10 g dotierten Träger aus Beispiel 1 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) und Ni(NO₃)₂*6H₂O (567 mg, 1,95 mmol) in 8,3 g Wasser (pH = 2,3) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 8,64.

### Beispiel 25

Eine Suspension von 10 g dotierten Träger aus Beispiel 11 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 9,69.

### Beispiel 26

Eine Suspension von 10 g dotierten Träger aus Beispiel 12 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 9,29.

### Beispiel 27

Eine Suspension von 10 g dotierten Träger aus Beispiel 13 in 33,3 g VE Wasser wurde auf 90 °C aufgeheizt und 15 min bei dieser Temperatur gerührt. Zu dieser Suspension wurde unter Rühren eine vorher auf 90 °C vorgeheizte Lösung von HAuCl₄*3H₂O (205 mg) in 8,3 g Wasser (pH = 1,6) zugegeben. Nach der Zugabe wurde das Gemisch weitere 30 min gerührt, dann abgekühlt und bei Raumtemperatur filtriert und anschließend sechsmal mit jeweils 50 mL Wasser gewaschen. Das Material wurde 10 h bei 105 °C getrocknet, fein gemörsert, innerhalb von 1h von 18 auf 450 °C aufgeheizt und schließlich bei 450 °C für 5h kalziniert. PZC von diesem Material war 9,14.

### Beispiele im Batchverfahren zur MMA-Herstellung

Ein goldhaltiger Katalysator (384 mg), Methacrolein (1,20 g) und Methanol (9,48g) wurden 2 h bei 60 °C und 30 bar Druck in einer Atmosphäre von 7 vol% O₂ in N₂ in einem 140 mL Stahlautoklaven mit Magnetrüher gerührt. Nach 2 h wurde das Gemisch abgekühlt, entgast, filtriert und mittels Gaschromatographie (GC) analysiert. Jeder Katalysator wurde mindestens zweimal unter identischen Bedingungen getestet und die Ergebnisse der jeweiligen Experimente gemittelt. Der resultierende Umsatz von Methacrolein (U(MAL) in %), die Raum-Zeit-Ausbeute (RZA, angegeben in mol MMA/kg Kat-h) und die Selektivität der Umsetzung des Methacroleins zu MMA (S(MMA), in %) für jeden getesteten Katalysator sind in der folgenden Tabelle 1 zusammengefasst.

**Tabelle 1 (Katalysatorperformance in Batchtests nach der Herstellung):**

| **Bsp.** | **Dotierung** | **PZC (Kat)** | **PZC (Träger)** | **pH (Au Lösung)** | **U(MAL), %** | **RZA, mol MMA/kg Kat-h** | **S(MMA), %** |
|---|---|---|---|---|---|---|---|
| VB2 | Sn | 8,89 | 6,85 | 1,6 | 20,7 | 3,8 | 85,3 |
| 14 | Bi | 9,09 | 9,21 | 1,6 | 42,5 | 8,2 | 91,4 |
| 15 | Mn | 8,72 | 8,95 | 1,6 | 49,4 | 9,4 | 89,7 |
| 16 | Ce | 8,92 | 9,56 | 1,6 | 65,0 | 13,2 | 95,5 |
| 17 | Zr | 8,87 | 8,90 | 1,6 | 37,6 | 7,3 | 90,3 |
| 18 | Pb | 8,59 | 9,35 | 1,6 | 62,4 | 12,5 | 94,2 |
| 19 | Al | 8,82 | 9,12 | 1,6 | 30,7 | 6,3 | 93,1 |
| VB3 | Mg | 11,15 | 9,81 | 12,0 | 20,1 | 3,6 | 92,3 |
| 20 | Mg | 9,49 | 9,81 | 1,6 | 41,7 | 8,5 | 98,6 |
| 21 | Mg | 8,57 | 9,36 | 1,6 | 29,5 | 6,3 | 91,9 |
| VB4 | Mg | 6,99 | 7,91 | 1,6 | 21,2 | 3,9 | 82,3 |
| 22 | Mg | 9,29 | 9,81 | 0,4 | 31,9 | 6,5 | 85,3 |
| 23 | Mg | 9,63 | 9,81 | 5,2 | 34,8 | 7,1 | 92,5 |
| 24 | Y | 8,80 | 9,34 | 1,6 | 43,7 | 8,7 | 92,8 |
| VB5 | Ni | 8,64 | 9,81 | 2,3 | 74,9 | 15,4 | 99,0 |
| 25 | La | 9,69 | 10,20 | 1,6 | 48,8 | 10,1 | 95,7 |
| 26 | Mg | 9,29 | 12,14 | 1,6 | 31,6 | 5,5 | 81,4 |
| 27 | Li | 9,14 | 12,31 | 1,6 | 29,2 | 5,2 | 81,9 |

Mit diesen Beispielen und Vergleichsbeispielen konnte gezeigt werden, dass Katalysatoren mit einem zu hohen PZC-Wert (VB3) oder einem zu geringen PZC-Wert (VB4) eine gegenüber vergleichbaren Katalysatoren mit einem erfindungsgemäßen PZC-Wert (Beispiele 20 und 21) geringere Aktivität, in Form einer verminderten Raum-Zeit-Ausbeute (RZA) haben. Auch konnte gezeigt werden, dass eine Reihe von Metallen - wie beansprucht - gute Aktivitäten und Selektivitäten zeigen, während zum Beispiel wider Erwarten Zinn (VB2) weniger geeignet ist und damit nicht erfindungsgemäß als Komponente d) verwendet werden kann.

Auch konnte gezeigt werden, dass erfindungsgemäße Katalysatoren, die jedoch nicht nach dem besonders bevorzugten gleichsam erfindungsgemäßen Verfahren hergestellt wurden, gegenüber erfindungsgemäß bevorzugt hergestellten Katalysatoren schlechtere Eigenschaften aufweisen. So war bei den Beispielen 26 und 27 der PZC-Wert des Trägermaterials erfindungsgemäß zu hoch. Dies resultiert in beiden Fällen in zwar akzeptablen, jedoch nicht idealen Selektivitäten und Aktivitäten der Katalysatoren. Dieser Zusammenhang ergibt sich insbesondere aus einem Vergleich des Beispiels 26 mit dem Beispiel 20. Das gleiche gilt für den pH-Wert der goldhaltigen Lösung. So ergeben im Vergleich zum Beispiel 20 mit einem idealen Wert, die Katalysatoren, die mit einem zu tiefen (Beispiel 22) oder zu hohen (Beispiel 23) pH-Wert hergestellt wurden, gleichsam akzeptable, jedoch auch verbesserungswürdige Selektivitäten (Beispiel 22) bzw. Aktivitäten (Beispiele 22 und 23).

Die zunächst sehr gute Performance des aus der Literatur bekannten Ni-haltigen Katalysators gemäß VB5 relativiert sich zum Gegenteil, wenn man die Langzeitwirkung dieses Katalysators betrachtet (siehe dazu VB5, Tabelle 2).

### Hydrolysetests mit Goldkatalysatoren

### Beispiele 28 bis 32, Vergleichsbeispiele 6 und 7

4 g von einem Katalysatorpulver wurden in 200 g einer 10 gew%igen Natriumacetat-Lösung (pH = 7, eingestellt durch Zugabe von Essigsäure) unter Rühren (500 rpm) bei 80 °C suspendiert. Essigsäure wurde bei Bedarf zugegeben um pH = 7 konstant zu halten. Nach 16 h Rühren bei 80 °C wurde der Katalysator abfiltriert, mit VE Wasser gewaschen und bei 105 °C innerhalb von 10 h im Trockenschrank getrocknet. Der so behandelte (trockene) Katalysator wurde in einem Batchtest zur MMA-Herstellung wie vorher beschrieben getestet. Die Ergebnisse dieser Tests (Mittelwerte aus jeweils zwei Batch-Tests) sind in der folgenden Tabelle zusammengefasst und mit den Batch-Tests mit den frischen Katalysatoren verglichen.

**Tabelle 2 (Katalysatorperformance in Batchtests nach einem Hydrolysetest)**

| | | **Frischer Katalysator** | | | **Behandelter Katalysator** | | |
|---|---|---|---|---|---|---|---|
| **Bsp.** | **Kat Bsp.** | **U(MAL), %** | **RZA, mol MMA/kg Kat*h** | **S(MMA), %** | **U(MAL), %** | **RZA, mol MMA/kg Kat*h** | **S(MMA), %** |
| VB6 | VB5(Ni) | 74,9 | 15,4 | 99,0 | 8,5 | 1,1 | 59,9 |
| 28 | 16(Ce) | 65,0 | 13,2 | 95,5 | 46,3 | 8,9 | 91,6 |
| 29 | 25(La) | 48,8 | 10,1 | 95,7 | 56,2 | 10,8 | 92,8 |
| 30 | 18(Pb) | 62,4 | 12,5 | 94,2 | 55,9 | 11,2 | 93,5 |
| 31 | 20(Mg) | 41,7 | 8,5 | 98,6 | 40,1 | 8,3 | 93,8 |
| VB7 | 22(Mg) | 31,9 | 6,5 | 85,3 | 23,6 | 4,8 | 85,6 |
| 32 | 23(Mg) | 34,8 | 7,1 | 92,5 | 35,1 | 6,9 | 87,6 |

Mit diesen Versuchen konnte gezeigt werden, dass die Langzeitwirkung der erfindungsgemäßen Katalysatoren sehr gut ist, während Katalysatoren nach Stand der Technik, die nicht erfindungsgemäße Komponenten d), wie z.B. Ni, enthalten (VB5) einen sehr starken Wirkungsabfall zeigen und unter kontinuierlichen Produktionsbedingungen schnell wieder ausgetauscht werden müssten. Das gleiche gilt gemäß Vergleichsbeispiel 7 für Katalysatoren mit einem nicht optimalen pH Wert der goldhaltigen Lösung bei der Katalysatorherstellung. Auch diese leiden unter einem hohen Aktivitätsverlust in einem wässrigen Medium.

### Kontinuierlicher Test zur Herstellung von MMA (allgemeine Beschreibung)

In einen Rührkesselreaktor mit einem Gesamtvolumen von 400 ml wurden 20 g Pulverkatalysator vorgelegt. Der pH-Wert einer 42,5 gew%igen Lösung von MAL in Methanol wurde durch die Zugabe einer 1 gew%igen Lösung NaOH in Methanol unter Rühren auf pH = 7 eingestellt. Diese Lösung wurde mit einer konstanten Zugaberate kontinuierlich in den gerührten und begasten Rührkesselreaktor (Begasung mit Luft) unter 10 bar Druck und 80 °C Innentemperatur zugeführt. Gleichzeitig wurden in diesen Reaktor so viel 1 gew%ige NaOH-Lösung (in Methanol) zugeführt, dass der Wert im Reaktor bei pH = 7 konstant blieb. Das Reaktionsgemisch wurde über einen Filter aus dem Reaktor kontinuierlich entnommen. Die Produktproben wurden nach unten angegebener Zeit entnommen und gaschromatographisch analysiert.

### Vergleichsbeispiel 8 Konti-Test zur Herstellung von MMA mit Au/NiO Katalysator

In diesem Beispiel wurden 20 g von dem NiO-Au Katalysator aus dem Vergleichsbeispiel 5 (hergestellt analog EP2177267A1 und EP2210664A1) in der kontinuierlichen MMA-Herstellung, wie vorher beschrieben, eingesetzt. Die Ergebnisse sind in der Tabelle 3 zusammengefasst.

### Beispiel 33

In diesem Beispiel wurden 20 g von einem erfindungsgemäßen CeO₂-Au Katalysator aus dem Beispiel 15 in der MMA-Herstellung wie vorher beschrieben eingesetzt. Die Ergebnisse sind in der Tabelle 3 zusammengefasst.

**Tabelle 3. Kontinuierliche MMA-Herstellung mit ausgewählten Katalysatoren.**

| **Bsp.** | **TOS [h]** | **Feed [g/h]** | **U(MAL), %** | **RZA, mol MMA/kg Kat*h** | **S(MMA), %** |
|---|---|---|---|---|---|
| VB5 | 100 | 47,0 | 77,6 | 10,4 | 96,1 |
| VB5 | 1000 | 47,0 | 69,7 | 9,3 | 91,5 |
| 33 | 100 | 55,6 | 68,2 | 10,1 | 96,0 |
| 33 | 1000 | 55,0 | 62,5 | 9,9 | 96,0 |

Mit dem Vergleich in einer kontinuierlich durchgeführten oxidativen Veresterung kann schließlich gezeigt werden, dass gegenüber dem Ni-haltigen Katalysator, wie er im Stand der Technik beschrieben ist, die Selektivität eines erfindungsgemäßen Katalysators nach 1000 h Betriebsdauer auf konstant hohem Niveau bleibt, während der Katalysator des Standes der Technik 4,6% Selektivität verliert. Auch die Aktivität des erfindungsgemäßen Katalysators geht in diesem Zeitraum deutlich weniger zurück.

## Patentansprüche

1. Katalysator in Form von Partikeln für die oxidative Veresterung von Aldehyden zu Carbonsäureestern, **dadurch gekennzeichnet, dass** der Katalysator mit Ausnahme von Sauerstoff aus_a) 0,01 bis 10 mol% Gold, b) 40 bis 94 mol% Silizium, c) 3 bis 40 mol% Aluminium und d) 2 bis 40 mol% mindestens eines weiteren Elements ausgewählt aus Alkalimetallen, Erdalkalimetallen, Lanthanoiden mit den Ordnungszahlen 57 bis 71, Y, Sc, Ti, Zr, Cu, Mn, Pb und/oder Bi besteht, wobei die Komponenten b) bis d) als Oxide vorliegen und sich die Mengenangaben der Komponenten a) bis d) auf 100 mol% der Zusammensetzung des Katalysators ohne Sauerstoff beziehen,
dass der Katalysator eine Schalenstruktur aus einem Kern und mindestens einer Schale aufweist, wobei mindestens 80 % der Gesamtmenge der Komponente a) Bestandteil einer Schale sind,
und dass der Katalysator einen PZC-Wert zwischen 7 und 11 aufweist, wobei der PZC-Wert wie in der Beschreibung beschrieben bestimmt wird.

2. Katalysator gemäß Anspruch 1, **dadurch gekennzeichnet, dass** der Katalysator zwischen 0,05 und 2 mol% der Komponente a) aufweist.

3. Katalysator gemäß Anspruch 1 oder 2, **dadurch gekennzeichnet, dass** die Komponente a) in Form von Partikeln mit einem mittleren Durchmesser zwischen 2 und 10 nm vorliegt.

4. Katalysator gemäß mindestens einem der Ansprüche 1 bis 3, **dadurch gekennzeichnet, dass** die Katalysator-Partikel einen mittleren Durchmesser zwischen 10 und 200 µm und eine sphärische Form aufweisen.

5. Katalysator gemäß mindestens einem der Ansprüche 1 bis 4, **dadurch gekennzeichnet, dass** der Katalysator zwischen 2 und 30 mol% Mg, Ce, La, Y, Zr, Mn, Pb und/oder Bi als Komponente d) aufweist.

6. Katalysator gemäß mindestens einem der Ansprüche 1 bis 5, **dadurch gekennzeichnet, dass** der Katalysator einen Kern und zwei Schalen aufweist, wobei mindestens 80 % der Gesamtmenge der Komponente a) sich in der inneren Schale befinden.

7. Verfahren zur Herstellung von Katalysator-Partikeln gemäß einem der Ansprüche 1 bis 6, **dadurch gekennzeichnet, dass** ein basischer Mischoxid-Träger mit einem PZC-Wert zwischen 8 und 12 und einem mittleren Durchmesser zwischen 10 und 200 µm, bestehend aus b) 40 bis 94 mol% Silizium, c) 3 bis 40 mol% Aluminium und d) 2 bis 40 mol% mindestens eines weiteren Elements ausgewählt aus Alkalimetallen, Erdalkalimetallen, Lanthanoiden mit den Ordnungszahlen 57 bis 71, Y, Sc, Ti, Zr, Cu, Mn, Pb und/oder Bi, wobei die Komponenten b) bis d) als Oxide vorliegen und sich die Mengenangaben der Komponenten a) bis d) auf 100 mol% der Zusammensetzung des Katalysators ohne Sauerstoff beziehen, mit einer goldhaltigen Lösung zur Bildung einer Komponente a), aufweisend einen pH-Wert zwischen 0,5 und 5,0 versetzt wird.

8. Verfahren gemäß Anspruch 7, **dadurch gekennzeichnet, dass** die goldhaltige Lösung mindestens eine zusätzliche Verbindung, aufweisend Komponenten b), c) und/oder d) in ionischer Form, enthält.

9. Verwendung eines Katalysators gemäß mindestens einem der Ansprüche 1 bis 6 bei der Umsetzung von (Meth)acrolein mit Sauerstoff und einem monofunktionellen Alkohol zu einem Alkyl(meth)acrylat.

10. Verwendung gemäß Anspruch 9, **dadurch gekennzeichnet, dass** Methacrolein mit Sauerstoff und Methanol zu Methylmethacrylat (MMA) umgesetzt wird.

11. Verwendung eines Katalysators gemäß mindestens einem der Ansprüche 1 bis 6 bei der Umsetzung von (Meth)acrolein mit Sauerstoff und einem di-, tri- oder tetra-funktionellen Alkohol zu einem Hydroxyalkyl(meth)acrylat oder zu einem di-, tri- bzw. tetra-(Meth)acrylat.

12. Verwendung gemäß Anspruch 9 bis 11, **dadurch gekennzeichnet, dass** die oxidative Veresterung kontinuierlich durchgeführt wird.

13. Verwendung gemäß Anspruch 12, **dadurch gekennzeichnet, dass** der Katalysator während der oxidativen Veresterung in einem gerührten Reaktor in Suspensionsform vorliegt.

## Claims

1. Catalyst in the form of particles for the oxidative esterification of aldehydes to carboxylic esters, **characterized in that** the catalyst consists, except for oxygen, of a) 0.01 to 10 mol% of gold, b) 40 to 94 mol% of silicon, c) 3 to 40 mol% of aluminium and d) 2 to 40 mol% of at least one further element selected from alkali metals, alkaline earth metals, lanthanoids having atomic numbers 57 to 71, Y, Sc, Ti, Zr, Cu, Mn, Pb and/or Bi, where components b) to d) are present as oxides and the stated amounts of components a) to d) relate to 100 mol% of the composition of the catalyst without oxygen,
**in that** the catalyst has a shell structure composed of a core and at least one shell, where at least 80% of the total amount of component a) is part of a shell,
and **in that** the catalyst has a PZC value between 7 and 11, where the PZC value is determined as described in the description.

2. Catalyst according to Claim 1, **characterized in that** the catalyst includes between 0.05 and 2 mol% of component a).

3. Catalyst according to Claim 1 or 2, **characterized in that** component a) is in the form of particles having an average diameter between 2 and 10 nm.

4. Catalyst according to at least one of Claims 1 to 3, **characterized in that** the catalyst particles have an average diameter between 10 and 200 µm and a spherical shape.

5. Catalyst according to at least one of Claims 1 to 4, **characterized in that** the catalyst includes between 2 and 30 mol% of Mg, Ce, La, Y, Zr, Mn, Pb and/or Bi as component d).

6. Catalyst according to at least one of Claims 1 to 5, **characterized in that** the catalyst has a core and two shells, wherein at least 80% of the total amount of component a) is within the inner shell.

7. Process for producing catalyst particles according to any of Claims 1 to 6, **characterized in that** a basic mixed oxide support having a PZC value between 8 and 12 and an average diameter between 10 and 200 µm, consisting of b) 40 to 94 mol% of silicon, c) 3 to 40 mol% of aluminium and d) 2 to 40 mol% of at least one further element selected from alkali metals, alkaline earth metals, lanthanoids having atomic numbers 57 to 71, Y, Sc, Ti, Zr, Cu, Mn, Pb and/or Bi, where components b) to d) are present as oxides and the stated amounts of components a) to d) relate to 100 mol% of the composition of the catalyst without oxygen, is admixed with a gold-containing solution to form a component a) having a pH between 0.5 and 5.0.

8. Process according to Claim 7, **characterized in that** the gold-containing solution contains at least one additional compound including components b), c) and/or d) in ionic form.

9. Use of a catalyst according to at least one of Claims 1 to 6 in the reaction of (meth)acrolein with oxygen and a monofunctional alcohol to give an alkyl (meth)acrylate.

10. Use according to Claim 9, **characterized in that** methacrolein is reacted with oxygen and methanol to give methyl methacrylate (MMA).

11. Use of a catalyst according to at least one of Claims 1 to 6 in the reaction of (meth)acrolein with oxygen and a di-, tri- or tetrafunctional alcohol to give a hydroxyalkyl (meth)acrylate or to give a di-, tri-or tetra(meth)acrylate.

12. Use according to Claims 9 to 11, **characterized in that** the oxidative esterification is conducted continuously.

13. Use according to Claim 12, **characterized in that** the catalyst is present in suspension form in a stirred reactor during the oxidative esterification.

## Revendications

1. Catalyseur sous forme de particules pour l'estérification oxydante d'aldéhydes en esters d'acides carboxyliques, **caractérisé en ce que** le catalyseur est constitué, outre l'oxygène, a) de 0,01 à 10 % en moles d'or, b) de 40 à 94 % en moles de silicium, c) de 3 à 40 % en moles d'aluminium et d) de 2 à 40 % en moles d'au moins un élément supplémentaire choisi parmi des métaux alcalins, des métaux alcalino-terreux, des lanthanoïdes dotés des numéros atomiques 57 à 71, Y, Sc, Ti, Zr, Cu, Mn, Pb et/ou Bi, les composants b) à d) étant présents en tant qu'oxydes et les données quantitatives des composants a) à d) se rapportant à 100 % en moles de la composition du catalyseur sans l'oxygène,
**en ce que** le catalyseur présente une structure de coque composée d'un noyau et d'au moins une enveloppe, au moins 80 % de la quantité totale du composant a) faisant partie d'une enveloppe,
et **en ce que** le catalyseur présente une valeur PZC comprise entre 7 et 11, la valeur PZC étant déterminée comme décrit dans la description.

2. Catalyseur selon la revendication 1, **caractérisé en ce que** le catalyseur comprend entre 0,05 et 2 % en moles du composant a).

3. Catalyseur selon la revendication 1 ou 2, **caractérisé en ce que** le composant a) est présent sous forme de particules dotées d'un diamètre moyen compris entre 2 et 10 nm.

4. Catalyseur selon au moins l'une des revendications 1 à 3, **caractérisé en ce que** les particules de catalyseur présentent un diamètre moyen compris entre 10 et 200 µm et présentent une forme sphérique.

5. Catalyseur selon au moins l'une des revendications 1 à 4, **caractérisé en ce que** le catalyseur présente entre 2 et 30 % en moles de Mg, Ce, La, Y, Zr, Mn, Pb et/ou Bi en tant que composant d).

6. Catalyseur selon au moins l'une des revendications 1 à 5, **caractérisé en ce que** le catalyseur présente un noyau et deux enveloppes, au moins 80 % de la quantité totale du composant a) se trouvant dans l'enveloppe interne.

7. Procédé pour la préparation de particules de catalyseur selon l'une quelconque des revendications 1 à 6, **caractérisé en ce qu'**un support d'oxyde mixte basique doté d'une valeur de PZC comprise entre 8 et 12 et d'un diamètre moyen compris entre 10 et 200 µm, constitué b) de 40 à 94 % en moles de silicium, c) de 3 à 40 % en moles d'aluminium et d) de 2 à 40 % en moles d'au moins un élément supplémentaire choisi parmi des métaux alcalins, des métaux alcalino-terreux, des lanthanoïdes dotés des numéros atomiques 57 à 71, Y, Sc, Ti, Zr, Cu, Mn, Pb et/ou Bi, les composants b) à d) étant présents en tant qu'oxydes et les données quantitatives des composants a) à d) se rapportant à 100 % en moles de la composition du catalyseur sans l'oxygène, est mis à réagir avec une solution contenant de l'or pour la formation d'un composant a), présentant une valeur de pH comprise entre 0,5 et 5,0.

8. Procédé selon la revendication 7, **caractérisé en ce que** la solution contenant de l'or contient au moins un composé supplémentaire, présentant les composants b), c) et/ou d) sous forme ionique.

9. Utilisation d'un catalyseur selon au moins l'une des revendications 1 à 6 lors de la transformation de (méth)acroléine avec de l'oxygène et un alcool monofonctionnel en un (méth)acrylate d'alkyle.

10. Utilisation selon la revendication 9, **caractérisée en ce que** la méthacroléine est transformée avec de l'oxygène et du méthanol en méthacrylate de méthyle (MMA) .

11. Utilisation d'un catalyseur selon au moins l'une des revendications 1 à 6 lors de la transformation de (méth)acroléine avec de l'oxygène et un alcool difonctionnel, trifonctionnel ou tétrafonctionnel en un (méth)acrylate d'hydroxyalkyle ou un di(méth)acrylate, tri(méth)acrylate ou tétra(méth)acrylate.

12. Utilisation selon les revendications 9 à 11, **caractérisée en ce que** l'estérification oxydante est mise en œuvre de manière continue.

13. Utilisation selon la revendication 12, **caractérisée en ce que** le catalyseur est présent, pendant l'estérification oxydante dans un réacteur agité, sous forme de suspension.
